# EUROPEAN PATENT APPLICATION

(11) **EP 4 681 770 A1**
(43) Date of publication of application: **21.01.2026**
(21) Application number: 24854267.2
(22) Date of filing: 28.05.2024
(51) Int. Cl.: A61N 2/02, A61N 2/00, H01F 5/02, H01F 17/02

(54) **MAGNETISM GENERATION COIL THAT PROVIDES NON-INVASIVE TREATMENT**

(30) Priority: 17.08.2023 KR 20230107336
(71) Applicant: Remed Brainstim Co., Ltd., Seongnam-si Gyeonggi-do 13647 (KR)
(72) Inventor: YOON, Se Jin, Anyang-si, Gyeonggi-do 14055 (KR); CHO, Sang Hee, Seoul 01799 (KR); KIM, Il Kyu, Suwon-si, Gyeonggi-do 16354 (KR); KIM, Young Kwang, Seoul 01318 (KR); CHOI, Ji Ho, Ansan-si, Gyeonggi-do 15350 (KR); JO, Jang Won, Seoul 06308 (KR)
(74) Representative: ABG Intellectual Property Law, S.L.
(86) International application number: PCT/KR2024/007195
(87) International publication number: WO 2025/037714

(57) **Abstract**

The present invention relates to a magnetic generation coil that provides non-invasive treatment by applying a magnetic field to nerve cells or muscle cells of the body. The magnetic generation coil of the present invention comprises: a coil bundle consisting of a collection of a plurality of unit coil strands of a copper material; and a hollow tube surrounding the coil bundle and formed of a flexible material. The unit coil strands forming the magnetic generation coil of the present invention may be formed of an oxygen-free copper (OFC) coil having a low ratio of oxygen to copper.

## Description

### TECHNICAL FIELD

The present invention relates to a magnetic generation coil, and more particularly, to a magnetic generation coil that provides non-invasive treatment by applying a magnetic field to nerve cells or muscle cells of the body.

### BACKGROUND ART

Transcranial magnetic stimulation (TMS) is a brain stimulation technique that uses local magnetic field waves induced on a surface of a head outside a human body to activate or suppress nerve cells in specific areas of a brain. The principle of the TMS is to apply strong electric current to an electromagnetic coil, thereby generating a magnetic field of a degree of several Tesla. This is a method for stimulating the brain by transmitting fluctuating energy of the magnetic field waves to the brain and inducing depolarization in nerve cells within a range of several centimeters below the coil.

The TMS is a representative non-invasive brain stimulation technique, and its therapeutic application is being actively studied in depression, insomnia, obsessive-compulsive disorder, movement disorders, dementia, and brain dysfunction, which do not respond well to drug treatment and psychotherapy.

Neuromagnetic stimulation (NMS) or pulsed electromagnetic field (PEMF) treatment is a method used to treat inflammation, swelling, and pain caused by damage of soft tissues such as skin, muscles, tendons, and ligaments due to musculoskeletal disorders. An electromagnetic field is directly transmitted to the damaged tissue to induce microcurrents, thereby promoting recovery of the damaged cells.

In addition, core-muscle strength magnetic stimulation (CSMS) is a method for strengthening core muscles by applying high-frequency magnetic stimulation to repeat muscle contraction and relaxation more than 40 times per second.

To perform the various types of magnetic stimulation treatments, a position of a stimulator including a coil having a certain diameter gradually moves to find a point at which the greatest stimulation occurs. Thereafter, a stimulation intensity is determined based on a threshold so that a corresponding portion is repeatedly stimulated.

Conventionally, development of a structure of increasing in strength of a magnetic field and focusing the magnetic field on a desired target area has continued through structural designs of various magnetic generation coils. Since the electrical activity induced from the coil is highly focused and attenuated very quickly away from a center of the coil, coil design technologies and the development of coil technology with a structure for proper contact with the target area are important for optimal stimulation.

### DISCLOSURE OF THE INVENTION

### TECHNICAL PROBLEM

An object of the present invention is to provide a magnetic generation coil having a structure for proper contact with a target area and effective focusing of a magnetic field.

In addition, an object of the present invention is to provide a magnetic generation coil having a structure and material, which minimize factors that interrupt a flow of current in consideration of an impedance of the coil.

In addition, an object of the present invention is to provide a flexible magnetic generation coil so that an applicator including the magnetic generation coil of the present invention is formed to be fitted into curves of various body portions.

### TECHNICAL SOLUTION

A magnetic generating coil of the present invention to achieve the above objects, which applies a magnetic field to nerve cells or muscle cells of a human body to provide non-invasive treatment, includes: a coil bundle formed by gathering a plurality of unit coil strands made of a copper material; and a hollow tube made of a flexible material and configured to surround the coil bundle.

In addition, the magnetic generation coil of the present invention may be a disc-shaped coil in which the coil bundle and the hollow tube are wound to be integrated with each other.

In addition, in the magnetic generation coil of the present invention, the coil bundle may include a first coil bundle and a second coil bundle connected in parallel to the first coil bundle, and the hollow tube may include a first tube configured to surround the first coil bundle and a second tube configured to surround the second coil bundle.

In addition, in the magnetic generation coil of the present invention, the disc-shape coil may be formed in a two-layer structure, and the first coil bundle and the second coil bundle may form a first layer and a second layer of the disc-shaped coil, respectively.

In addition, in the magnetic generation coil of the present invention, the first tube and the second tube may be in close contact with each other so that at least portions of the first tube and the second tube are connected to each other.

In addition, the magnetic generation coil of the present invention may further include: an input-side coil bundle connected to a first end of the first coil bundle and a first end of the second coil bundle; and an output-side coil bundle connected to a second end of the first coil bundle and a second end of the second coil bundle.

In addition, in the magnetic generation coil of the present invention, diameters of the input-side coil bundle and the output-side coil bundle may be larger than diameters of the first coil bundle and the second coil bundle.

In addition, in the magnetic generation coil of the present invention, the unit coil bundle may be formed as an oxygen free copper (OFC) coil having a low oxygen-to-copper ratio.

### ADVANTAGEOUS EFFECTS

As described above, the magnetic generation coil of the present invention may be formed with the flexible structure and material and thus may have the advantage of being able to achieve the proper contact with the target area and the effective focusing of the magnetic field.

In addition, the magnetic generation coil of the present invention may be formed so that the applicator including the magnetic generation coil of the present invention is formed to be fitted into the curves of the various body portions.

In addition, the magnetic generation coil of the present invention may use the oxygen free copper (OFC) coil, which minimizes the elements that interrupt the flow of the current, to reduce the impedance of the coil, thereby reducing the heat generated from the coil and maximizing the magnetic generation efficiency.

In addition, the magnetic generation coil of the present invention may have the advantage of being able to implement the coil assembly capable of controlling the heat generation without coupling the separate cooling means by using the oxygen free copper (OFC) coil.

### BRIEF DESCRIPTION OF THE DRAWINGS

FIG. 1 is a perspective view of a magnetic generation coil according to an embodiment of the present invention.
FIG. 2 is a cross-sectional view taken along line A-A' in the magnetic generation coil according to an embodiment of the present invention.
FIG. 3 is a cross-sectional view taken along line B-B' in the magnetic generation coil according to an embodiment of the present invention.
FIG. 4 is a cross-sectional view taken along line C-C' in the magnetic generation coil according to an embodiment of the present invention.
FIG. 5 is a schematic view for explaining a structure of the magnetic generation coil according to an embodiment of the present invention.
FIG. 6 is a graph obtained by comparing results of a heat generation temperature of a magnetic generation coil that is another type of magnetic generation coil according to an embodiment of the present invention.

### MODE FOR CARRYING OUT THE INVENTION

Advantages and features of the present disclosure, and implementation methods thereof will be clarified through following examples described with reference to the accompanying drawings. The present invention may, however, be embodied in different forms and should not be construed as limited to the embodiments set forth herein. Rather, these embodiments are provided so that this disclosure will be thorough and complete, and will fully convey the scope of the present invention to those skilled in the art. Further, the present invention is only defined by scopes of claims.

Although the terms first, second, etc. are used to describe various components, these components are not limited by these terms. These terms are only used to distinguish one component from another component. Accordingly, a first component that will be described below may be a second component within the technical idea of the present disclosure.

In the embodiments below, the terms such as "include" or "have" mean that a feature or component described in the specification is present, and do not exclude in advance the possibility that one or more other features or components may be added.

In the drawings, for convenience of description, the dimensions of elements are exaggerated or downscaled. For example, since the size and shape of each of the components illustrated in the drawings are arbitrarily shown for convenience of description, the present invention is not necessarily limited to the illustrated.

Like reference numerals refer to like elements throughout.

Each feature of the various embodiments of the present invention can be partially or fully coupled or combined with each other, and as can be fully understood by those skilled in the art, various technical interconnections and operations are possible. Also, the embodiments may be independently performed with respect to each other or performed in combination of each other.

Hereinafter, a magnetic generation coil 100 of the present invention will be described in detail with reference to the attached drawings.

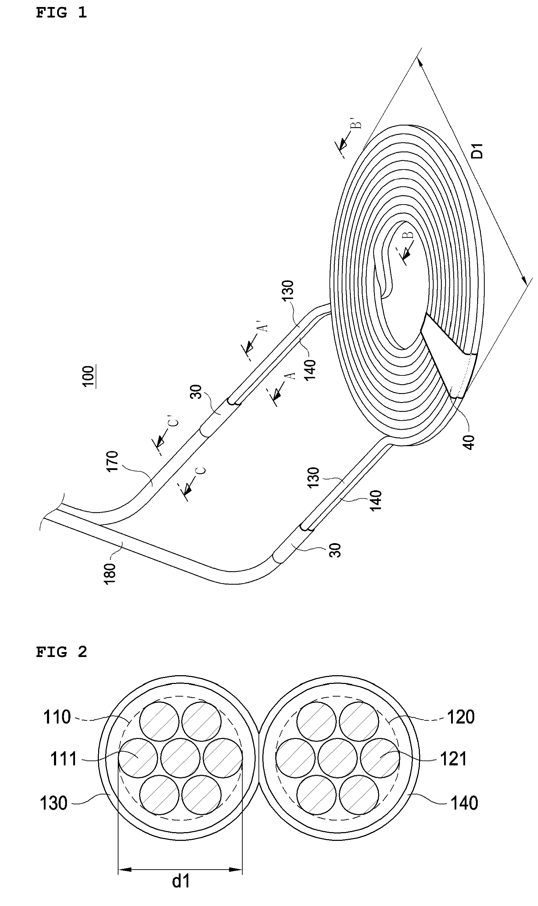

FIG. 1 is a perspective view of the magnetic generation coil 100 according to an embodiment of the present invention.

The magnetic generation coil 100 of the present invention is a coil that generates a magnetic field and applies the magnetic field to nerve or muscle cells of the body to provide non-invasive treatment.

The magnetic generation coil 100 of the present invention is a magnetic generation coil that is capable of being used for transcranial magnetic stimulation (TMS), neuromagnetic stimulation (NMS), pulsed electromagnetic stimulation (PEMF), or core muscle magnetic stimulation (CSMS).

Hereinafter, a structure of the magnetic generation coil 100 of the present invention will be described in more detail with reference to FIGS. 2 to 5, which specifically illustrate a cross-section of each portion of FIG. 1.

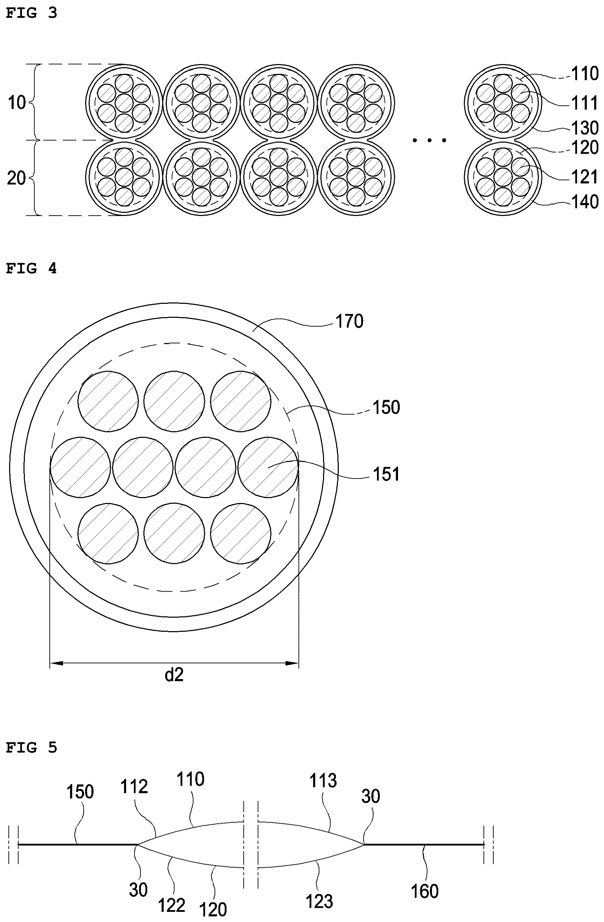

FIG. 2 is a cross-sectional view taken along line A-A' in the magnetic generation coil according to an embodiment of the present invention, FIG. 3 is a cross-sectional view taken along line B-B' in the magnetic generation coil according to an embodiment of the present invention, FIG. 4 is a cross-sectional view taken along line C-C' in the magnetic generation coil according to an embodiment of the present invention, and FIG. 5 is a schematic view for explaining a structure of the magnetic generation coil according to an embodiment of the present invention.

Referring to FIG. 2, the magnetic generation coil 100 of the present invention includes coil bundles 110 and 120 including unit coil strands 111 and 121 made of a copper material, and hollow tubes 130 and 140 surrounding the coil bundles 110 and 120. The magnetic generation coil 100 of the present invention may be a disc-shaped coil in which the coil bundles 110 and 120 and the hollow tubes 130 and 140 are wound to be integrated with each other.

The coil bundles 110 and 120 include a first coil bundle 110 and a second coil bundle 120 connected in parallel to the first coil bundle 110.

The coil bundles 110 and 120 is provided by gathering a plurality of unit coil strands 111 and 121. The first coil bundle 110 is provided by gathering a plurality of unit coil strands 111, and the second coil bundle 120 is provided by gathering a plurality of unit coil strands 121.

Here, it is preferable that each of the unit coil strands 111 and 121 is provided as an oxygen free copper (OFC) coil having a low oxygen-to-copper ratio.

The OFC coil means a high-purity copper wire with a purity of 99.9% or higher, containing no oxides other than copper. The higher the oxygen content in the coil, the more likely it is that the current flow is interrupted. Conversely, the higher the copper purity, the smoother the current flow. The OFC coil used in the magnetic generation coil 100 of the present invention has a lower oxygen ratio than that of a general coil and thus has lower resistance or impedance, so the magnetic generation coil 100 of the present invention has an advantage of low heat generation.

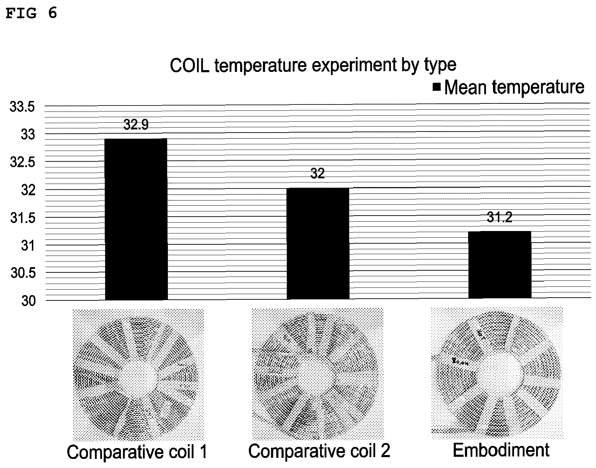

It is preferable that a coil selected between AWG 8 and AWG 12, which is a standard specification of a wire diameter (or cross-sectional area) is used as the OFC coil used in the magnetic generation coil 100 of the present invention. When the OFC coil having a diameter within the above-described range is selected and used, it may be confirmed that heat generation is controlled without separate cooling while performing the non-invasive treatment using the magnetic generation coil 100 of the present invention. Specific heat control performance will be described later with reference to FIG. 6.

The hollow tubes 130 and 140 include a first tube 130 that surrounds the first coil bundle 110 and a second tube 140 that surrounds the second coil bundle 120.

The first tube 130 surrounds the first coil bundle 110, and the second tube 140 surrounds the second coil bundle 120. The hollow tubes 130 and 140 may be PVC tubes made of a flexible material. The hollow tubes 130 and 140 serve to encapsulate the coil bundles 110 and 120.

Here, the first tube 130 and the second tube 140 may be in close contact with each other and be at least partially connected to each other. As illustrated in FIG. 2, in the first tube 130 and the second tube 140, outer circumferences of the first tube 130 and the second tube 140 may be partially connected to each other so that a cross-section thereof has a figure-8 shape. The first tube 130 and the second tube 140 may be formed in a form in which the first tube 130 and the second tube 140 are in close contact with each other in a longitudinal direction.

The magnetic generation coil 100 of the present invention may be a disc-shaped coil in which the coil bundles 110 and 120 and the hollow tubes 130 and 140 are wound to be integrated with each other, as illustrated in FIG. 1.

Here, a diameter D1 of the magnetic generation coil 100 may be 100 mm to 300 mm. More preferably, the first diameter D1 may be 150 mm to 250 mm.

The disc-shaped magnetic generation coil 100 may be formed in a two-layer structure. Referring to FIG. 3, the magnetic generation coil 100 includes a first layer 10 and a second layer 20. The first coil bundle 110 and the second coil bundle 120 described above may form the first layer 10 and the second layer 20 of the disc-shaped coil, respectively.

The magnetic generation coil 100 of the present invention further includes an input-side coil bundle 150 and an output-side coil bundle 160.

The first coil bundle 110 forming the first layer 10 and the second coil bundle 120 forming the second layer 20 of the disc-shaped magnetic generation coil 100 may be connected in parallel to the input-side coil bundle 150 and the output-side coil bundle 160.

Referring to FIG. 5, the input-side coil bundle 150 is connected to a first end 112 of the first coil bundle 110 and a first end 122 of the second coil bundle 120. The output-side coil bundle 160 is connected to the second end 113 of the first coil bundle 110 and the second end 123 of the second coil bundle 120.

Here, it is preferable that a diameter d2 of the input-side coil bundle 150 is larger than the diameter D1 of the first coil bundle 110.

In addition, the diameter of the output-side coil bundle 160 may be the same as the diameter d2 of the input-side coil bundle 150, and the diameter of the second coil bundle 120 may be the same as the diameter D1 of the first coil bundle 110.

A connection part 30 may be provided at a point connecting the input-side coil bundle 150 to the first coil bundle 110 and the second coil bundle 120 and/or at a point connecting the output-side coil bundle 160 to the first coil bundle 110 and the second coil bundle 120. The connection part 30 electrically connects the input-side coil bundle 150, the first coil bundle 110, and the second coil bundle 120 to each other.

The input-side tube 170 surrounds the input-side coil bundle 150. The input-side tube 170 may be a hollow tube like the first tube 130 and the second tube 140, and may be a flexible PVC tube. The hollow input-side tube 170 serves to encapsulate the input-side coil bundle 150. Likewise, an output-side tube 180 may be provided, and the output-side tube 180 surrounds the output-side coil bundle 160.

The magnetic generation coil 100 of the present invention may further include a support part 40. Referring to FIG. 1, the support part 40 serves to support the disc-shaped coil in which the coil bundles 110 and 120 and the hollow tubes 130 and 140 are wound to be integrated with each other. The support part 40 may be a belt-shaped member capable of simultaneously surrounding and supporting coils, which are wound in a plurality of layers, of the magnetic generation coil 100.

The conditions of current, voltage, frequency, etc., applied to the magnetic generation coil 100 of the present invention are exemplified as follows.
Current = 500 ~ 900 A (P-P)
Voltage = 100 ~ 1000V (P-P)
Frequency = 1 ~ 100 Hz

However, it should be noted that these figures are exemplary and do not limit the scope of the present invention.

FIG. 6 is a graph obtained by comparing results of a heat generation temperature of a magnetic generation coil that is another type of magnetic generation coil according to an embodiment of the present invention.

An illustrated comparative coil 1 is a solid-type copper coil used in the conventional magnetic generation coil, and a comparative coil 2 is a coil that has a shape in which a coil bundle formed by gathering a plurality of unit coil strands is wound, like the coil of the present invention, but is made of a tin material. The coil of the embodiment corresponds to the magnetic generation coil 100 of the present invention.

The comparative coil 1, the comparative coil 2, and the coil according to the embodiment have the same conductor diameter of 12AWG, and the wound disc has an inner diameter of Ø90 and an outer diameter of Ø300. At room temperature of 26°C, current was applied to each coil, and temperatures were measured three times after approximately one hour. A shown temperature represents a mean value obtained through the three measurements.

While a temperature of the comparative coil 1 and a temperature of the comparative coil 2 rapidly increased to 32.9°C and 32°C, respectively, the magnetic generation coil 100 of the present invention showed a lower temperature rise of 31.2°C compared to the comparative coil 1 and the comparative coil 2.

A temperature difference of approximately 1°C as described above may have a significant effect on heat control performance. Since magnetic treatment is performed for more than 20 minutes using the magnetic generation coil, the magnetic generation coil 100 of the present invention, which shows the lower temperature rise compared to the comparative coils, may reduce heat generated from the coil during that magnetic treatment lasting several tens of minutes and maximize magnetic generation efficiency.

In the above, the magnetic generation coil 100 according to an embodiment of the present invention is exemplified as having the overall circular shape, but is not limited thereto. The magnetic generation coil 100 may be a coil wound to have various shapes such as an oval, figure 8, disc, or polygon when viewed from the vertical upper or lower portions.

Although the embodiment of the inventive concept is described with reference to the accompanying drawings, those with ordinary skill in the technical field of the inventive concept pertains will be understood that the present disclosure can be carried out in other specific forms without changing the technical idea or essential features. Therefore, the above-disclosed embodiments are to be considered illustrative and not restrictive.

This project (outcome) is results of the local government-university collaboration-based regional innovation project that is supported by the National Research Foundation of Korea and funded by the Ministry of Education in 2023 (2021RIS-001).

This research was supported by "Regional Innovation Strategy (RIS)" through the National Research Foundation of Korea (NRF) funded by the Ministry of Education (MOE) (2021RIS-001)

## Claims

1. A magnetic generation coil that applies a magnetic field to nerve cells or muscle cells of a human body to provide non-invasive treatment, the magnetic generation coil comprising:
a coil bundle formed by gathering a plurality of unit coil strands made of a copper material; and
a hollow tube made of a flexible material and configured to surround the coil bundle.

2. The magnetic generation coil of claim 1, wherein the magnetic generation coil is a disc-shaped coil in which the coil bundle and the hollow tube are wound to be integrated with each other.

3. The magnetic generation coil of claim 2, wherein the coil bundle comprises a first coil bundle and a second coil bundle connected in parallel to the first coil bundle, and
the hollow tube comprises a first tube configured to surround the first coil bundle and a second tube configured to surround the second coil bundle.

4. The magnetic generation coil of claim 3, wherein the disc-shaped coil is formed in a two-layer structure, and
the first coil bundle and the second coil bundle form a first layer and a second layer of the disc-shaped coil, respectively.

5. The magnetic generation coil of claim 3, wherein the first tube and the second tube are in close contact with each other so that at least portions of the first tube and the second tube are connected to each other.

6. The magnetic generation coil of claim 3, further comprising:
an input-side coil bundle connected to a first end of the first coil bundle and a first end of the second coil bundle; and
an output-side coil bundle connected to a second end of the first coil bundle and a second end of the second coil bundle.

7. The magnetic generation coil of claim 6, wherein diameters of the input-side coil bundle and the output-side coil bundle are larger than diameters of the first coil bundle and the second coil bundle.

8. The magnetic generation coil of claim 1, wherein the unit coil strand is formed as an oxygen free copper (OFC) coil having a low oxygen-to-copper ratio.
